(19) Europäisches Patentamt
European Patent Office
Office européen des brevets

(11) Publication number: **0 181 706**
**B1**

(12) **EUROPEAN PATENT SPECIFICATION**

(45) Date of publication of patent specification: **15.11.89**

(51) Int. Cl.$^4$: **C 07 F 5/00,** C 23 C 16/18

(21) Application number: **85307400.3**

(22) Date of filing: **15.10.85**

(54) **Hybrid organometallic compounds of In and ba and process for metal organic chemical vapour deposition.**

(30) Priority: **25.10.84 US 664645**

(43) Date of publication of application:
**21.05.86 Bulletin 86/21**

(45) Publication of the grant of the patent:
**15.11.89 Bulletin 89/46**

(84) Designated Contracting States:
**AT BE CH DE FR GB IT LI NL SE**

(56) References cited:
**US-A-2 880 115**
**US-A-2 969 382**
**US-A-2 987 534**
**US-A-3 578 494**

**DICTIONARY OF ORGANOMETALLIC COMPOUNDS, vol. 1, Chapman and Hall, Londen, GB**

(73) Proprietor: **MORTON THIOKOL, INC.**
**110 North Wacker Drive**
**Chicago Illinois 60606-1560 (US)**

(72) Inventor: **Hui, Benjamin C.**
**25 Castle Circle**
**Peabody, MA 01960 (US)**
Inventor: **Lorberth, Jorg**
**Kiefernweg 3**
**D-3556 Weimar-Niederweimar (DE)**
Inventor: **Melas, Andreas A.**
**2 Pine Avenue**
**Burlington, MA 01803 (US)**

(74) Representative: **Bankes, Stephen Charles Digby et al**
**BARON & WARREN 18 South End Kensington**
**London W8 5BU (GB)**

(56) References cited:
**JOURNAL OF THE AMERICAN CHEMICAL SOCIETY, vol. 97, no. 7, 2nd April 1975, pages 1672-1679, Washington, D.C., US; A.D. CARDIN: "Cadmium-113 Fourier transform nuclear magnetic resonance spectroscopy"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 181 706 B1**

(56) References cited:
JOURNAL OF ORGANICAL CHEMISTRY, vol. 46, 1981, pages 4127-4130, American Chemical Society, Washington, D.C., US; A.D. BAKER et al.: "Photoelectron spectra of alkyl aryl selenides, electronic and steric factors in the observation of rotamers"

CHEMICAL ABSTRACTS, vol. 99, no. 25, 19th December 1983, page 654, no. 212623z, Columbus, Ohio, US; A.J. ASHE et al.: "Preparation and properties of dibismuthines", & ORGANOMETALLICS 1983, 2(12), 1859-66 Comprehensive organometallic chemistry, vol. 1, (Pergamon Press), pp. 588-590, 688

E. Wiberg "Neuere Ergebnisse der präperativen Hydridforschung", Angewandte Chemie 65 Jahrgang Nr. 1, Jan. 1953, p. 25

Stadelhofer, J. et al., J. Organomet. Chem., 1975, 84, C1-C4

Stadelhofer, J. et al., J. Organomet. Chem., 1976, 116, 65-73

IBM Technical Disclosure, vol. 17, no. 10, March 1975, p. 3096

**Description**

This invention relates to organometallic compounds comprising gallium and/or indium and mixed alkyl substituents. This invention also relates to metal organic chemical vapour deposition (MOCVD) processes such as are employed for example in the optoelectronics industry.

MOCVD is a method for depositing thin metal or metal compound films on a silicon or other substrate. The deposited films can be sources of doping impurities which are driven into the substrate, or the films themselves can have electrical or optical properties different from those of the substrate. These films are used to make laser diodes, solar cells, photocathodes, field effect transistors and other discrete devices, in fiber optic communications, microwave communications, digital audio disc systems, and other advanced optoelectronic technologies. The properties of the film depend on the deposition conditions and the chemical identity of the deposited film.

A special advantage of MOCVD is that organometallic compounds can be found which have much higher vapour pressure at moderate temperatures than the corresponding metals, and which decompose to release the corresponding metals or form compounds thereof at the 550 to 700 degrees Celsius deposition temperatures which should not be exceeded during fabrication.

Typical apparatus currently in use for MOCVD comprises a bubbler which contains a supply or the organometallic compound chosen for a particular process, a reactor or deposition chamber which contains the substrate on which a film is to be deposited, a source of a carrier gas which is inert to the organometallic compound in the bubbler and either inert or reactive to the compound in the deposition chamber, and optionally sources of other reactants or dopants supplied to the reaction chamber. The bubbler and contents are maintained at a constant and relatively low temperature which typically is above the melting point of the organometallic compound but far below its decomposition temperature. The deposition chamber is typically maintained at a much higher temperature, such as about 550 to 700 degrees Celsius, at which the organometallic compound readily decomposes to release its constituent metal. To operate the MOCVD apparatus, the carrier gas is introduced into the bubbler under the surface of the organometallic compound. Rising bubbles of the carrier gas provide a large, constant contact surface and thus uniformly vapourize the organometallic compound. The carrier gas and vapour collected in the headspace of the bubbler are continuously directed to the deposition chamber.

An example of such apparatus, and its use, is disclosed in IBM Technical Disclosure Bulletin Vol. 17 No. 10 of March 1975, page 3096. A further example of such apparatus, with multiple compound sources enabling a chemical reaction between different source compounds within the deposition chamber, is disclosed in US—A—4105810.

While it is possible to vapourize a sublimable solid organometallic compound in a bubbler, it is difficult to control its rate of vapourization. The surface area of a solid exposed to the carrier gas changes as vapourization proceeds. In contrast, a liquid contained in a bubbler with substantially vertical walls presents the same surface area of a solid to the carrier gas so long as the flow and bubble size of the carrier gas remains steady. Thus, organometallic compounds for MOVCD desirably are liquids at or slightly above room temperature (about −20°C to 40°C). Such compounds also should have a vapour pressure of at least 133.3 Pa (1.0 torr) at the bubbler temperature, boil and decompose at temperatures substantially exceeding the bubbler temperature, and decompose readily at the temperature encountered in the deposition chamber.

A number of organometallic compounds of aluminium, boron, cadmium, mercury, thallium and zinc, with two different organic substituent groups, are disclosed in the Dictionary of Organometallic compounds, vol. 1, Chapman and Hall, London. Also disclosed therein are organohydride compounds of benyllium, magnesium and gallium, and alkyl/cyclopentadienyl gallium compounds.

Further such organo boron compounds are disclosed in US—A—2969382, organozinc compounds in US—A—3578494 and organocadmium compounds in JACS vol. 97 No. 7, PP 1672 to 1679.

Organoselenium compounds with two different alkyl or anyl substituents are disclosed in J. Org. Chem. Vol. 46, 1981, pp 4127—4130. (Am. Chem. Soc., Washington DC, USA).

Organobismuth compounds with two different alkyl substituents are disclosed in Chem. Abs., Chemical Substance Index, A—B, Vol. 99 pt.1., 1983 p. 1319 CS col. 2.

Bis (cyclopentadienyl) (methylcyclopentadienyl) aluminium is disclosed in US—A—2987534.

Cyclopentadienyl dimethyl aluminium is disclosed in Comprehensive Organometallic Chemistry, Vol. 1. (Pergamor Press) pp 588—590 and 688.

Finally, methods of gas plating organoaluminium and organomagnesium compounds are disclosed in US—A—2880115.

For certain metals, notably gallium and indium it is difficult to select a useful candidate having the necessary properties for MOCVD.

Another factor complicates the selection of a workable organometallic compound for MOCVD: structurally related organometallic compounds often do not form homologous series. Many organometallic compounds characteristically exist in only one form, for example, as monomers, dimers, trimers, tetramers, or higher polymers. Structurally similar compounds often have different characteristic forms, and thus much different or inconsistent vapour pressure, melting points, and decomposition temperatures.

As particular case in point, consider the two known compounds of indium—trimethylindium and triethylindium. Both of these compounds have been used to deposit Indium containing films. (See: 1. Manasevit and Simpson, *J. Electrochem. Soc.,* 118, C291 (1971): 120,135 (1973); 2 Bass, J. *Crystal Growth,* 31, 172 (1975) 3. Duchemin, et al., Paper 13, *7th Intern. Symp, on GaAs and Related Compounds,* Clayton, MD, Sept., 1978). Though they are structurally similar, the respective melting points, vapour pressures at 30 degrees Celsius and decomposition temperatures of these compounds are inconsistent with what would be expected of homologs, as illustrated by Table 1 below:

TABLE 1

| Property | Triethylindium | Trimethylindium |
|---|---|---|
| Melting point | −32°C | 88°C |
| Vapour pressure at 30°C | 0.8 torr (106.6 Pa) | 7.2 torr (959.8 Pa) |
| Decomposition temperatures | 40°C | 260°C |

Trimethylindium is believed to characteristically form a tetramer and triethylindium is believed to characteristically form a monomer at room temperature. This difference is believed to underline their inconsistent properties. The preceding table illustrates that trimethylindium is a solid at temperatures employed in bubblers, and has a vapour pressure of sublimation which is undesirably low. Trimethylindium has been vapourized by providing two bubblers in series to better control the amount of entrained vapour. The apparatus necessary for this two bubbler procedure is more expensive and complex, and yet provides less control of the partial pressure of trimethylindium, than apparatus used to vapourize a liquid from a single bubbler. Triethylindium has an even lower vapour pressure at 30 degrees Celsius than trimethylindium, and is also less thermally and chemically stable than trimethylindium. Triethylindium starts to decompose to Indium at 35 degrees Celsius, and at an even lower temperature in the presence of hydrogen—the typical carrier gas. The vapourization of triethylindium thus must take place at a temperature approaching its decomposition temperature, and even then the deposition rate is undesirably low. The lack of homology in these Indium compounds and the small number of known Indium compounds have prevented those of ordinary skill in the art from selecting an optimal compound for Indium MOCVD.

A first aspect of the invention is a genus of compounds useful for metal organic chemical vapour deposition, defined by the molecular formula:

$$MR_3$$

wherein each said R substituent is a $C_1$ to $C_4$ alkyl group and at least two of the R substituents are different. M is gallium or indium. Such compounds are known herein as mixed or hybrid organometallic compounds, and are believed to be novel.

A second aspect of the invention is a metal organic chemical vapour deposition process wherein there is used a compound having the formula $MR_3$ defined above.

The apparatus employed for metal organic chemical vapour deposition generally comprises a deposition chamber maintained at a temperature between the melting point and the decomposition temperature of the compound being deposited, at which the said compound has a vapour pressure which is useful for a particular deposition process. The apparatus further comprises a deposition chamber maintained at a temperature at least as high as the decomposition temperature of the compound. Practice of this process allows one to tailor the molecular structure of an organometallic compound of a desired metal to fit the required specifications of vapour pressure, melting point, boiling point and decomposition temperature which are necessary for successful or optimal practice of MOCVD.

Still another aspect of the invention is a process for synthesising hybrid organometallic compounds, comprising the steps of:

(A) selecting different first and second compounds, each having the formula:

$$MR'_3$$

wherein each said R′ substituent is $C_1$—$C_4$ alkyl and wherein M in each case represents gallium or indium, and

(B) mixing said first and second compounds together to form a product.

The hybrid compounds thus produced can also be used in MOCVD processes accordingly to the invention.

Preferred embodiments of the invention will now be described with reference to the accompanying drawings wherein:

Figure 1 is an infrared absorption spectrum of dimethylethylindium.

Figure 2 is a proton nuclear magnetic resonance spectrum of dimethylethylindium.

Figure 3 is an infrared absorption spectrum of diethylmethylindium.

Figure 4 is a proton nuclear magnetic resonance spectrum of diethylmethylindium.

Figure 5 is a proton nuclear magnetic resonance spectrum of trimethylindium, a prior art compound.

Figure 6 is a proton nuclear magnetic resonance spectrum of triethylindium, a prior art compound.

R substituents contemplated for use in the novel compounds are alkyl groups having from one to four carbon atoms, and specifically include methyl, ethyl, n-propyl, isopropyl, n-butyl, isobutyl, and t-butyl groups.

The generic invention includes gallium or indium combined with any two or more R substituents as previously defined. The genus is limited, however, by the requirement that at least two different R substituents must be associated with each metal atom.

Exemplary species contemplated herein include the following:

dimethylethylgallium
dimethylethylindium
diethylmethylindium.

Hybrid organometallic compounds can be produced by mixing organometallic compounds containing the respective substituents of the hybrid; by reacting a halogen substituted organometallic compound with an alkylating or arylating agent to add an unlike substituent; by reacting the metal for which an organometallic hybrid compound is desired with mixtures of organic halides; by substituting a more active metal for a less active metal in an organometallic hybrid compound of the less active metal; or by other means.

To practice the mixing method described in the preceding paragraph, first and second organometallic compounds are first selected. Each reactant has the formula:

$$MR'_3$$

in which each R′ of the first compound can be identical, and each R′ of the second compound can be identical, but at least one R′ substituent of the first compound is different than at least one R′ substituent of the second compound. In a preferred embodiment of the mixing method invention, the first and second compounds are mixed together and allowed to equilibrate at a temperature below the lower of the boiling points of the reactants and products, preferably from 0—30° Celsius. A nonreactive solvent such as benzene, hexane, ether, tetrahydrofuran, etc. is optional. The result of this exchange reaction will typically be a major proportion of a hybrid organometallic compound according to the invention, in which the several R substituents are present in roughly the same proportions as in the reaction mixture containing the first and second reactants. Minor proportions of the reactants and of other organometallic products may also be present. The desired product can be isolated by distillation, crystallization, or other well known processes. Alternatively, the product mixture can be used for MOCVD without isolating a pure hybrid product. The following equations illustrate reactions of this type in which stoichiometric proportions of the reactants provide a major proportion of the indicated product:

$$2(CH_3)_3In(s)+(C_2H_5)_3In(l)\rightarrow 3(CH_3)_2C_2H_5In(l)$$

$$2(C_2H_5)_3In(l)+(CH_3)_3In(s)\rightarrow 3(C_2H_5)_2CH_3In(l)$$

In the second synthetic method identified above, a halogenated organometallic compound having one of the desired alkyl substituents is reacted with an alkylating agent. The alkyl group of the alkylating agent then replaces the halogen substituent of the organometallic compound. Typical alkylating agents for use herein include such materials as methyllithium, ethylmagnesium bromide, or lithium aluminium hydride. Examples of these synthetic reactions are set forth in the two following equations:

$$(C_2H_5)_2InCl+CH_3Li\rightarrow CH_3(C_2H_5)_2In+LiCl$$

$$(CH_3)_2GaCl+LiAlH_4\rightarrow (CH_3)_2GaH+LiAlH_3Cl$$

Other methods ordinarily used in organometallic synthesis, such as those discussed on pages 345—348 and 365—366 of Roberts and Caserio, *Basic Principles of Organic Chemistry,* W. A. Benjamin Inc. (New York: 1964) can also be adopted to synthesize the hybrid organometallic compounds defined herein.

As indicated previously, the present compounds have utility as reactants in MOCVD. Preferred reactants for this utility have a melting point of less than about 30 degrees Celsius, have a vapour pressure of at least 1.0 torrs at a temperature within the bubbler temperature range of from about minus 20 degrees Celsius to about 40 degrees Celsius, are stable at the indicated bubbler temperatures but readily decompose at a deposition chamber temperature of from about 550 to about 700 degrees Celsius, and are inert at bubbler temperatures with respect to at least one carrier gas such as hydrogen, nitrogen or helium.

The present compounds also have utility for the preparation of other such compounds within the scope of the present invention. For example, a hybrid organometallic compound which does not have a desirable decomposition tempeature may be reacted with another organometallic compound to produce a new hybrid.

The present compounds also have utility in organic synthesis and as catalysts, for example in Ziegler-Natta processes.

The ultimate utility of these compounds, employed in MOCVD, is to provide a coating of the constituent metal, or (in combination with other reactants introduced in the deposition chamber) to provide coatings of metal oxides, nitrides, III—V compounds, and so forth. The metal hybrid organometallic compounds can also be used as dopants.

Process for selecting and using hybrid organometallic compounds for MOCVD

A second aspect of the present invention is a process for selecting particular hybrid organometallic compounds of gallium and/or indium which are useful in metal organic chemical vapour deposition processes.

The present process, characterised as an MOCVD process, comprises the steps of selecting first and second compounds, each having the formula $MR'_3$ as further defined below, making a hybrid compound having at least one substituent in common with the first compound and at least one substituent in common with the second compound, and employing the hybrid compound for metal organic chemical vapour deposition in apparatus further specified below. Since in the usual chemical vapour deposition process a particular element has been selected for deposition, usually the M constituents of the first and second compound will be the same. However, the process is not limited by this consideration nor has the second compound an optimal melting point, vapour pressure, or decomposition tempeature for use in MOCVD. First and second compounds meeting this definition are trimethylindium and triethylindium, whose melting points, volatility, and decomposition temperatures are set forth above in Table I above.

Other examples of first and second gallium or indium compounds useful in practicing the present invention can be found in the list of known homosubstituted and hybrid organometallic compounds in the "Background Art" section set forth previously.

Once first and second compounds have been selected, the next step is to make a composite compound having at least one R' substituent possessed by the first compound and at least one R' substituent possessed by the second compound. Although one manner of synthesizing the composite compound is by mixing the first and second compounds in the manner previously described, the present process is not limited to that method of synthesis. The other synthetic methods previously described, or method not specifically disclosed herein, can also be used within the scope of the present process invention. The composite compound should differ from each of the first and second compounds as to at least one property selected from decomposition temperature, vapour pressure at a particular temperature suitable for a bubbler, and melting point. By differing in respect to at least one of these properties, the composite compound will be useful for MOCVD under different process conditions than the first and second compounds. When one of the first and second compounds has a property such as melting point which is too low for a conventional MOCVD process and the other compound has a corresponding property which is too high for MOCVD, the composite compound defined herein may have a corresponding property between those of the first and second compounds or may have a surprisingly different value of the corresponding property.

Examples of the composite compounds within the scope of the second step of the process include the generic class and species set forth previously for the compound invention, as well as the previously mentioned hybrid organometallic compounds known to the art.

As a final step, the composite compound is employed for MOCVD in apparatus comprising a bubbler or equivalent apparatus maintained at a temperature between the melting point and decomposition temperature of the composite compound. The desired composite compound will have a vapour pressure at this temperature of at least 133.3 Pa (1.0 torr), and thus will be useful for deposition. The MOCVD apparatus used in this step further comprises a deposition chamber maintained at a temperature at least as high as the decomposition temperature of the composite compound, triggering the breakdown of the composite organometallic compound to release its constituent metal.

The present hybrid organometallic compounds have an advantage over any nonazeotropic mixture for use in MOCVD, as any nonazeotropic mixture will be fractionated by the carrier gas in a manner analogous to gas liquid chromatography.

Examples

The following examples are provided to further exemplify and demonstrate the use of the present compound and process inventions. The examples do not limit the scope of the invention, which is defined by the claims found at the end of the specification.

Example 1

Synthesis of dimethylethylindium

3.00 ml of triethylindium (3.78 g., 0.0187 mol) was added to 5.988 g (0.0374 mol) of trimethylindium in a 50 ml. flask in a glove bag under Argon atmosphere. The reagent were stirred at room temperature overnight. Reaction was essentially complete when all of the trimethylindium was fully reacted, leaving no residual solids. The resulting clear liquid was then distilled under full vacuum, (about 1.5 torrs pressure). Some of the resulting dimethylethylindium distilled over at room temperature, or about 23 degrees Celsius.

Gentle heating caused the rest to come over at 25 degrees Celsius, this temperature being measured at the distillation head. The resulting product had a melting point of about 5 to 7 degrees Celsius and a boiling point of 23—25°C at 200 Pa (1.5 torr), which is unexpectedly different than the respective melting points and boiling points of trimethylindium and triethylindium. Proton nuclear magnetic resonance and infrared spectra were taken, and are presented as Figure 1 and 2 forming a part of this specification. For comparison, the NMR spectra of trimethylindium and triethylindium are presented as Figures 5 and 6. The infrared spectrum is not believed to distinguish the product compound, but the NMR spectrum of dimethylethylindium is characterised by peaks at delta+1.27 (triplet representing ethyl); +0.37 (quartet representing ethyl); and −0.36 (singlet representing methyl). Integration of the areas under the peaks provides the ratio of methyl to ethyl groups, which is 2:1.

Example 2
Synthesis of diethylmethylindium

5.00 ml (6.30 g., 0.0312 mol) of triethylindium was added to 2.495 g (0.0156 mol) of trimethylindium in a 50 ml flask in a glove bag containing an Argon atmosphere. The mixture was stirred overnight and then distilled at 33 to 35 degrees Celsius under full vacuum as previously defined. The distillate was a clear, colourless liquid. NMR and IR spectra were taken, and are provided as Figures 3 and 4 herein. The NMR is characterized by peaks at delta values of +1.28 (triplet ethyl); +0.39 (quartet ethyl); and −0.39 (singlet methyl). An integration of the areas under the peaks shows a ratio of ethyl to methyl of 1.94:1. The melting point was found to be below about 3 degrees Celsius, as the product failed to solidify when the container was placed in ice water.

Example 3
Properties of trimethylindium and triethylindium (prior art)

Figure 5 is the NMR spectrum of trimethylindium, characterised by a singlet methyl peak at a delta value of −0.20. The melting point of trimethylindium is 88 degrees Celsius.

Figure 6 is the NMR spectrum of triethylindium, characterised by peaks at delta values of +1.24 (triplet ethyl) and +0.40 (quartet ethyl). The melting point of triethylindium is −32 degrees Celsius.

Example 4
Synthesis of other hybrid organometallic compounds

The procedure of Examples 1 and 2 is followed for the species previously listed herein. The reactants mixed to form the indicated species are provided below:

| Product | Reactant 1 | Reactant 2 |
|---|---|---|
| $(CH_3)_2GaC_2H_5$ | $(CH_3)_3Ga$ | $(C_2H_5)_3Ga$ |

Example 5
MOCVD process

Methyldiethylindium prepared as described previously is placed in a bubbler and suitably interconnected with a source of hydrogen gas and a deposition chamber. The chamber is also supplied with phosphine gas. The bubbler is maintained at 20 degrees Celsius using a suitable heat source, the deposition chamber is maintained at 650 degrees Celsius, and an Indium Phosphide substrate is supported within the deposition chamber. The entraining hydrogen is delivered at 100 cubic centimeters per minute (at standard temperature and pressure). The partial pressure of hydrogen in the deposition chamber is atmospheric pressure, and the partial pressure of methyldiethylindium is about 13.33 Pa (10 torr), the partial pressure of phosphine being atmospheric pressure. After about 30 minutes of deposition, a coating of indium phosphide approximately 2 µm thick, uniform in composition and thickness, is found to be deposited on the substrate.

**Claims for the Contracting States: BE, CH, DE, FR, GB, IT, LI, NL, SE**

1. A compound having the molecular formula:

$$MR_3$$

wherein each said R substituent is a $C_1$—$C_4$ alkyl group at least two of said R substituents are different, and M is gallium or indium.

2. A compound according to claim 1, wherein each R substituent is methyl or ethyl.
3. A compound according to claim 1 or claim 2, wherein at least one said R substituent is methyl.
4. A compound according to claim 3, wherein two said R substituents are methyl.
5. A compound according to any preceding claim, wherein at least one said R substituent is ethyl.
6. A compound according to claim 5, wherein two said R substituents are ethyl.

7. A compound according to any preceding claim, wherein M is Indium.

8. A compound according to claim 7, consisting essentially of dimethylethylindium or diethylmethylindium.

9. A compound according to claim 4 consisting essentially of dimethylethylgallium.

10. A process for synthesising hybrid organometallic compounds, comprising the steps of:

(A) selecting different first and second compounds, each having the formula:

$$MR'_3$$

wherein each said R' substituent is a $C_1$—$C_4$ alkyl and wherein M in each case represents gallium or indium, and

(B) mixing said first and second compounds together to form a product.

11. A process according to claim 10, comprising the further step of isolating said product.

12. A process according to claim 10 or claim 11 wherein said first and second compounds are mixed in stoichiometric proportions.

13. A metal organic chemical vapour deposition process wherein there is used a hybrid organometallic compound according to any one of claims 1 to 9 or one made by a process according to any one of claims 10 to 12.

**Claims for the Contracting State: AT**

1. A process for synthesising hybrid organometallic compounds, comprising the steps of:

(A) selecting different first and second compounds, each having the formula:

$$MR'_3$$

wherein each said R' substituent is a $C_1$—$C_4$ alkyl and wherein M in each case represents gallium or indium and

(B) mixing said first and second components together to form a product of formula:

$$MR_3$$

wherein each R substituent is a $C_1$—$C_4$ alkyl group, at least two of said R substituents are different and M is as defined above.

2. A process according to claim 1, wherein each R substituent is methyl or ethyl.

3. A process according to claim 1 or claim 2, wherein at least one said R substituent is methyl.

4. A process according to claim 3, wherein two said R substituents are methyl.

5. A process according to any preceding claim, wherein at least one said R substituent is ethyl.

6. A process according to claim 5, wherein two said R substituents are ethyl.

7. A process according to any preceding claim, wherein M is Indium.

8. A process according to claim 7, wherein the product consists essentially of dimethylethylindium or diethylmethylindium.

9. A process according to claim 4, wherein the product consists essentially of dimethylethylgallium.

10. A process according to any preceding claim comprising the further step of isolating said product.

11. A process according to any preceding claim wherein said first and second compounds are mixed in stoichiometric proportions.

12. A metal organic chemical vapour deposition process wherein there is used a hybrid organometallic compound made by a process according to any preceding claim.

**Patentansprüche für die Vertragsstaaten: BE, CH, DE, FR, GB, IT, LI, NL, SE**

1. Eine Verbindung mit der Molekularformel

$$MR_3$$

bei der jeder besagte Subtituent R eine $C_1$—$C_4$-Alkyl-Gruppe ist, mindestens zwei der besagten Substituenten R unterschiedlich sind und M Gallium oder Indium ist.

2. Eine Verbindung nach Anspruch 1, bei der jeder Substituent R Methyl oder Äthyl ist.

3. Eine Verbindung nach Anspruch 1 oder Anspruch 2, bei der mindestens einer der besagten Substituenten R Methyl ist.

4. Eine Verbindung nach Anspruch 3, bei der zwei de besagten Substituenten R Methyl sind.

5. Eine Verbindung nach einem der vorstehenden Ansprüche, bei der mindestens einer der besagten Substituenten R Äthyl ist.

6. Eine Verbindung nach Anspruch 5, bei der zwei der besagten Susbtituenten R Äthyl sind.

7. Eine Verbindung nach einem der vorstehenden Ansprüche, bei der M Indium ist.

8. Eine Verbindung nach Anspruch 7, die im wesentlichen aus Dimethyläthylindium oder Diäthylmethylindium besteht.

9. Eine Verbindung nach Anspruch 4, die im wesentlichen aus Dimethyläthylgallium besteht.

10. Ein Verfahren zum Synthetisieren hybrider Organometallverbindungen, umfassend die Schritte:

(A) der Wahl unterschiedlicher erster und zweiter Verbindungen, jede mit der Formel

$$MR'_3$$

worin jeder besagte Substituent R' ein $C_1$—$C_4$-Alkyl ist und worin M in jedem Falle in Gallium oder Indium besteht, und

(B) des Zusammenmischens der besagten ersten und zweiten Verbindungen, um ein Produkt zu bilden.

11. Ein Verfahren nach Anspruch 10, das den weiteren Schritt der Absonderung des besagten Produktes umfaßt.

12. Ein Verfahren nach Anspruch 10 oder Anspruch 11, bei dem die besagten ersten und zweiten Verbindungen in stöchiometrischen Proportionen gemischt werden.

13. Ein metallorganisches chemisches Bedampfungsverfahren, bei dem von einer hybriden Organometallverbindung nach einem der Ansprüche 1 bis 9 Gebrauch gemacht wird, oder von einer Verbindung, die nach einem Verfahren gemäß einem der Ansprüche 10 bis 12 hergestellt wurde.

**Patentansprüche für den Vertragstaat: AT**

1. Ein Verfahren zum Synthetisieren hybrider Organometallverbindungen, umfassend die Schritte:

(A) der Wahl verschiedener erster und zweiter Verbindungen, von denen jede die Formel

$$MR'_3$$

besitzt, worin jeder besagte Substituent R' ein $C_1$—$C_4$-Alkyl ist und worin M in jedem Falle in Gallium oder Indium besteht, und

(B) des Zusammenmischens der besagten ersten und zweiten Verbindungen, um ein Produkt der Formel

$$MR_3$$

zu bilden, worin jeder Substituent R eine $C_1$—$C_4$-Alkyl-Gruppe ist, wobei mindestens zwei der besagten Substituenten R unterschiedlich sind und M wie vorstehend definiert ist.

2. Ein Verfahren nach Anspruch 1, bei dem jeder Substituent R Methyl oder Äthyl ist.

3. Ein Verfahren nach Anspruch 1 oder Anspruch 2, bei dem mindestens einer der besagten Substituenten R Methyl ist.

4. Ein Verfahren nach Anspruch 3, bei dem zwei der besagten Substituenten R Methyl sind.

5. Ein Verfahren nach einem der vorstehenden Ansprüche, bei dem mindestens ein besagter Substituent R Äthyl ist.

6. Ein Verfahren nach Anspruch 5, bei dem zwei der besagten Substituenten R Äthyl sind.

7. Ein Verfahren nach einem der vorstehenden Ansprüche, bei dem M Indium ist.

8. Ein Verfahren nach Anspruch 7, bei dem das Produkt im wesentlichen aus Dimethyläthylindium oder Diäthylmethylindium besteht.

9. Ein Verfahren nach Anspruch 4, bei dem das Produkt im wesentlichen aus Dimethyläthylgallium besteht.

10. Ein Verfahren nach einem der vorstehenden Ansprüche, das den weiteren Schritt der Absonderung des besagten Produkts umfaßt.

11. Ein Verfahren nach einem der vorstehenden Ansprüche, bei dem die besagten ersten und zweiten Verbindungen in stöchiometrischen Proportionen gemischt werden.

12. Ein metallorganisches chemisches Bedampfungsverfahren, bei dem von einer hybriden Organometallverbindung Gebrauch gemacht wird, die nach einem auf einem der vorstehenden Ansprüche beruhenden Verfahren hergestellt wurde.

**Revendications pour les Etats Contractants: BE, CH, DE, FR, GB, IT, LI, NL, SE**

1. Un composé ayant la formule moléculaire:

$$MR_3$$

dont chacun desdits substituants R est une $C_1$—$C_4$ groupe alkyle, deux au moins desdits substituants R sont différents et M consiste de gallium ou d'indium.

2. Un composé selon la revendication 1, dont chaque substituant R est méthylique ou éthylique.

3. Un composé selon la revendication 1 ou 2, dont au moins un desdits substituants R est méthylique.

4. Un composé selon la revendication 3, dont deux desdits substituants R sont méthyliques.

5. Un composé selon toute revendication précédente, dont au moins un desdits substituants R est éthylique.

6. Un composé selon la revendication 5, dont deux desdits substituants R sont éthyliques.

7. Un composé selon toute revendication précédente, dont M consiste d'indium.

8. Un composé selon la revendication 7, consistant essentiellement de diméthyléthyl indium ou de diéthylméthyl indium.

9. Un composé selon la revendication 4, consistant essentiellement de diméthyléthyl gallium.

10. Procédé de synthèse des composès hybrides organométalliques, dont les phases comportent:

(A) la sélection d'un premier et deuxième composé différent, chacun ayant la formule:

$$MR'_3$$

dont chacun desdits substituants R' est un alkyle $C_1$—$C_4$ et dont M en chaque cas consiste de gallium ou d'indium, et

(B) le mélange desdits premier et deuxième composés pour former un produit.

11. Procédé selon la revendication 10, comportant une phase complémentaire d'isolement dudit produit.

12. Procédé selon la revendication 10 ou 11, dont lesdits premier et deuxième composés sont mélangés en proportions stoechiométriques.

13. Procédé de dépôt de vapeur chimique organique métallique exploitant un composé hybride organométallique selon l'une des revendications 1 à 9, ou un composé obtenu à partir d'un procédé selon l'une des revendications 10 à 12.

**Revendications pour l'Etat Contractant: AT**

1. Procédé de synthèse des composés hybrides organométalliques, comportant les phases:

(A) de sélection d'un premier et deuxième composé différent, chacun ayant la formule:

$$MR'_3$$

dont chacun desdits substituants R' est un alkyle $C_1$—$C_4$ et dont M en chaque cas consiste de gallium ou d'indium et

(B) de mélange desdits premier et deuxième composés pour former un produit ayant la formule:

$$MR_3$$

dont chaque susbtituant R est un groupe alkyle $C_1$—$C_4$, au moins deux desdits substituants R étant différents et M étant tel que défini ci-dessus.

2. Procédé selon la revendication 1, dont chaque substituant R est méthylique ou éthylique.

3. Procédé selon la revendication 1 ou 2, dont au moins un desdits substituants R est méthylique.

4. Procédé selon la revendication 3, dont deux desdits susbtituants R sont méthyliques.

5. Procédé selon toute revendication précédente, dont au moins un desdits substituants R est éthylique.

6. Procédé selon la revendication 5, dont deux desdits substituants R sont éthyliques.

7. Procédé selon toute revendication précédente, dont M consiste d'indium.

8. Procédé selon la revendication 7, dont le produit consiste essentiellement de diméthyléthyl indium ou de diéthylméthyl indium.

9. Procédé selon la revendication 4, dont le produit consiste essentiellement de diméthyléthyl gallium.

10. Procédé selon toute revendication précédente, comportant une phase complémentaire d'isolement dudit produit.

11. Procédé selon toute revendication précédente, dont lesdits premier et deuxième composés sont mélangés en proportions stoechiométriques.

12. Procédé de dépôt de vapeur chimique organique métallique exploitant un composé hybride organométallique obtenu par un procédé selon toute revendication précédente.

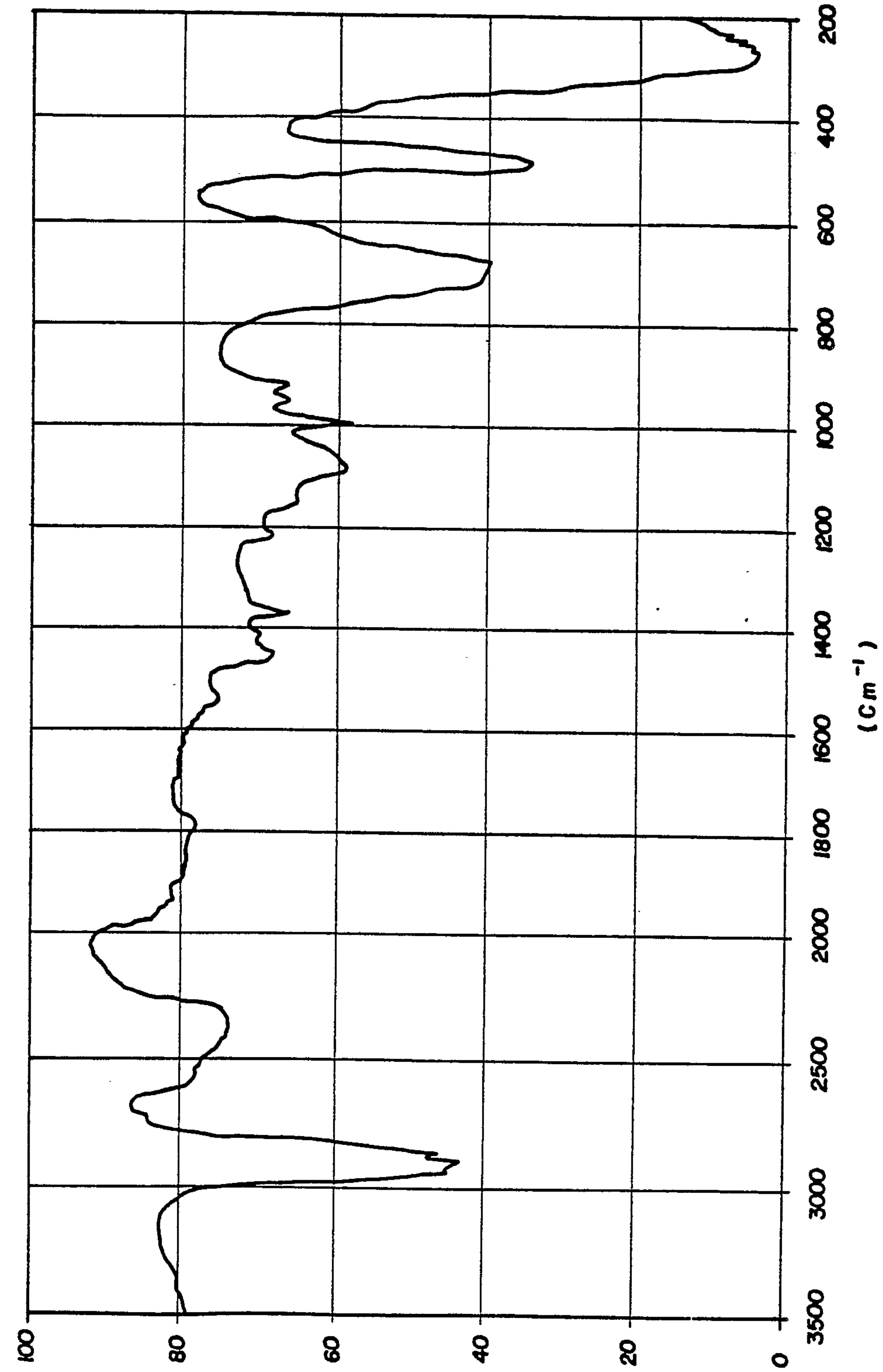

100
80
60
40
20
0
200
400
600
800
1000
1200
1400
1600
1800
2000
2500
3000
3500
(cm$^{-1}$)


Fig. 1

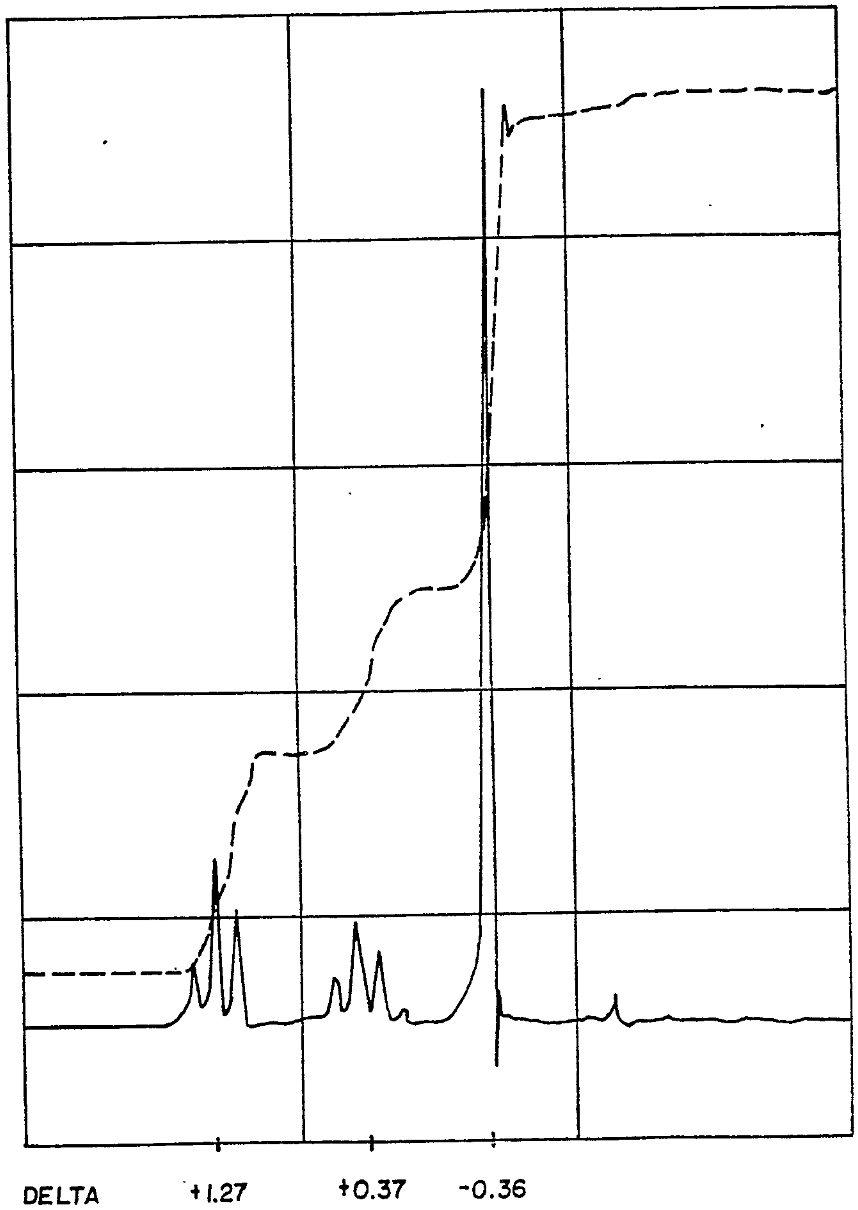


Fig. 2

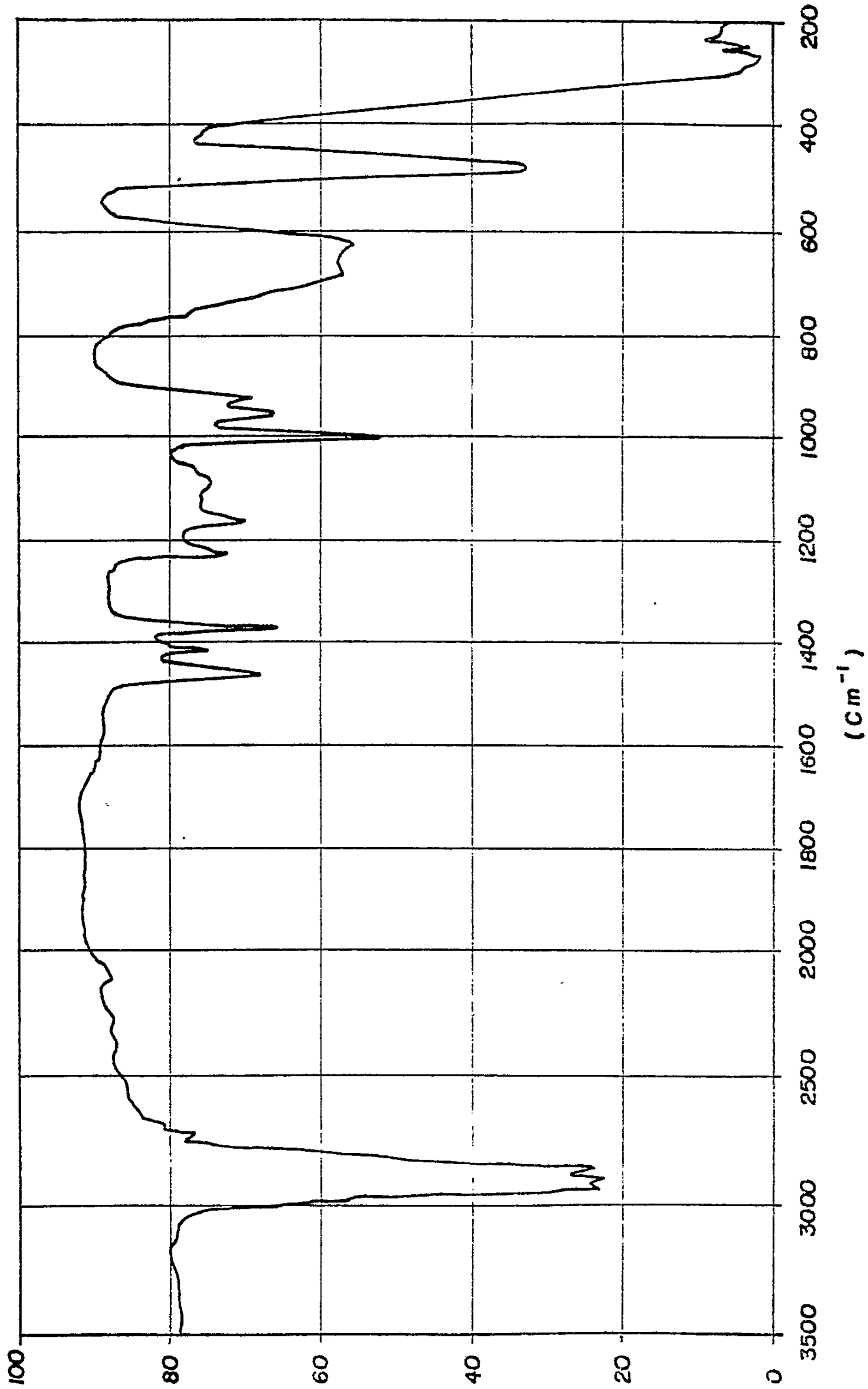
100
80
60
40
20
0
3500
3000
2500
2000
1800
1600
1400
1200
1000
800
600
400
200
(cm⁻¹)

Fig. 3

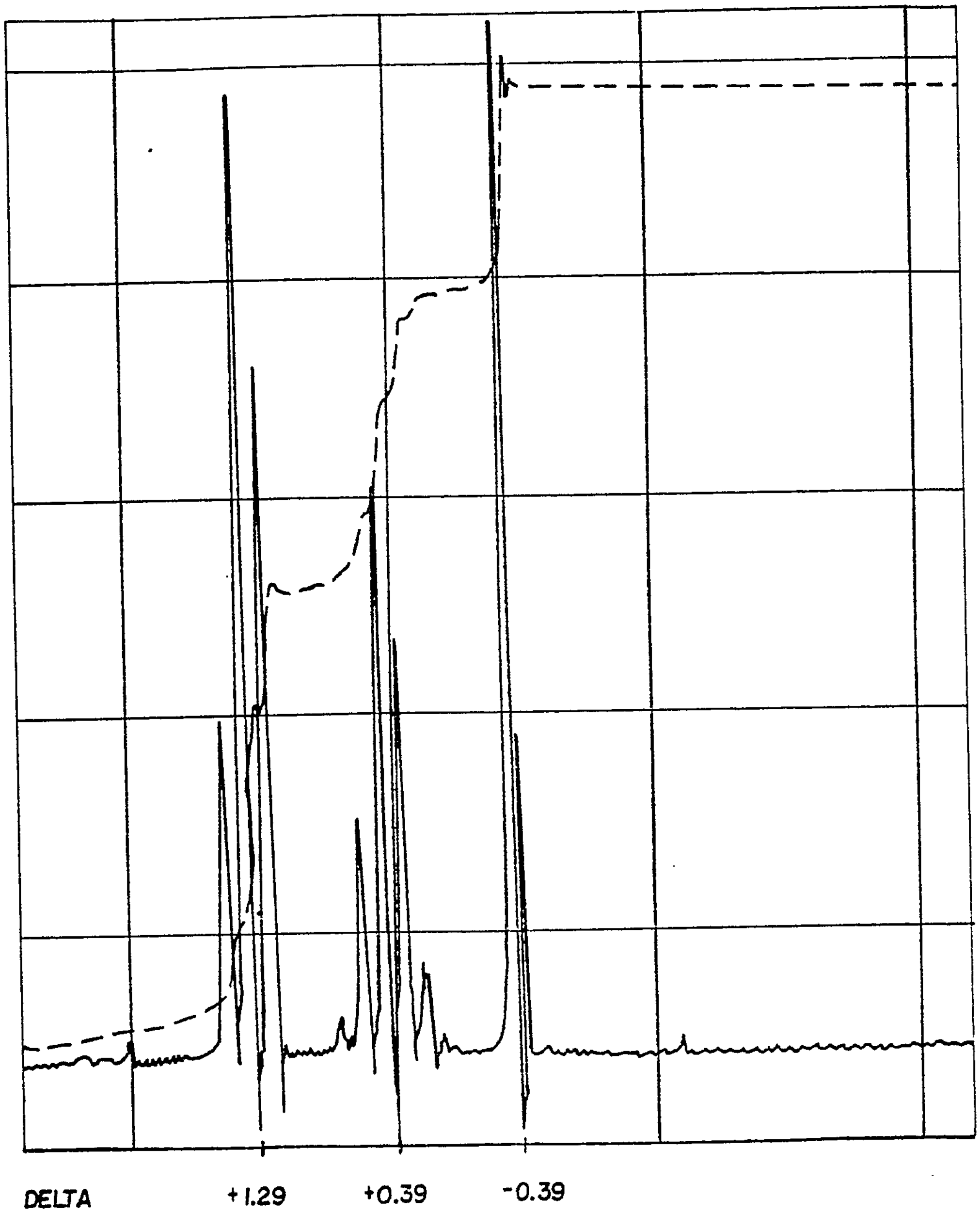


Fig. 4

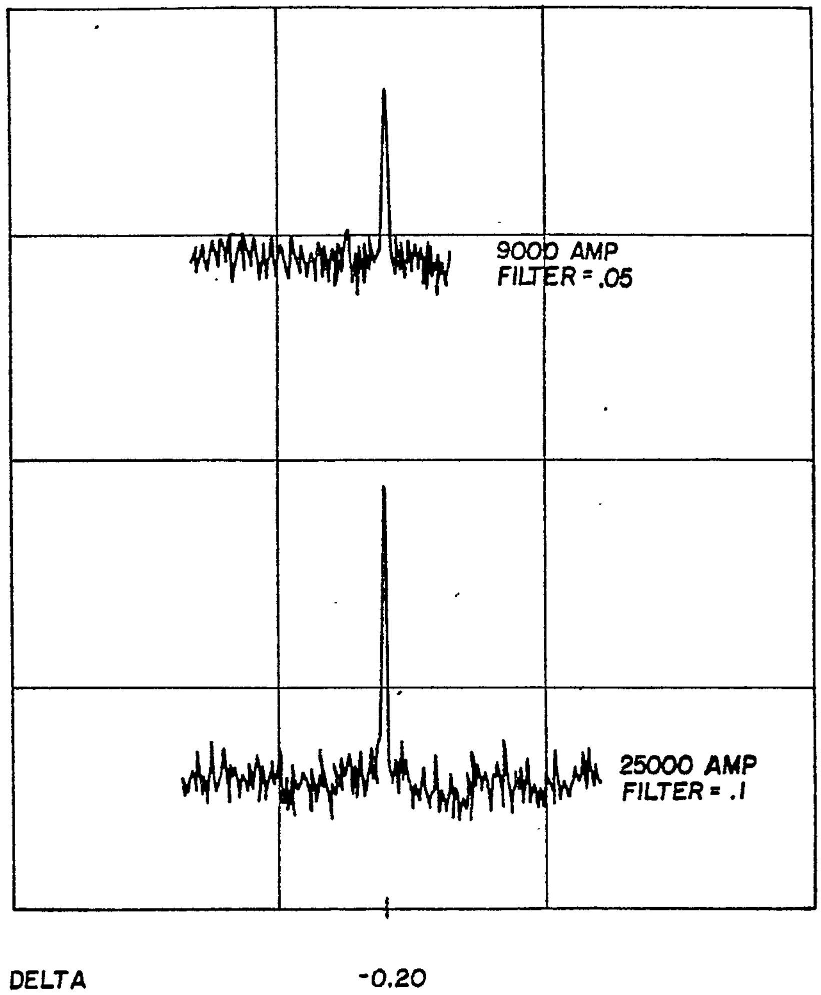


Fig. 5

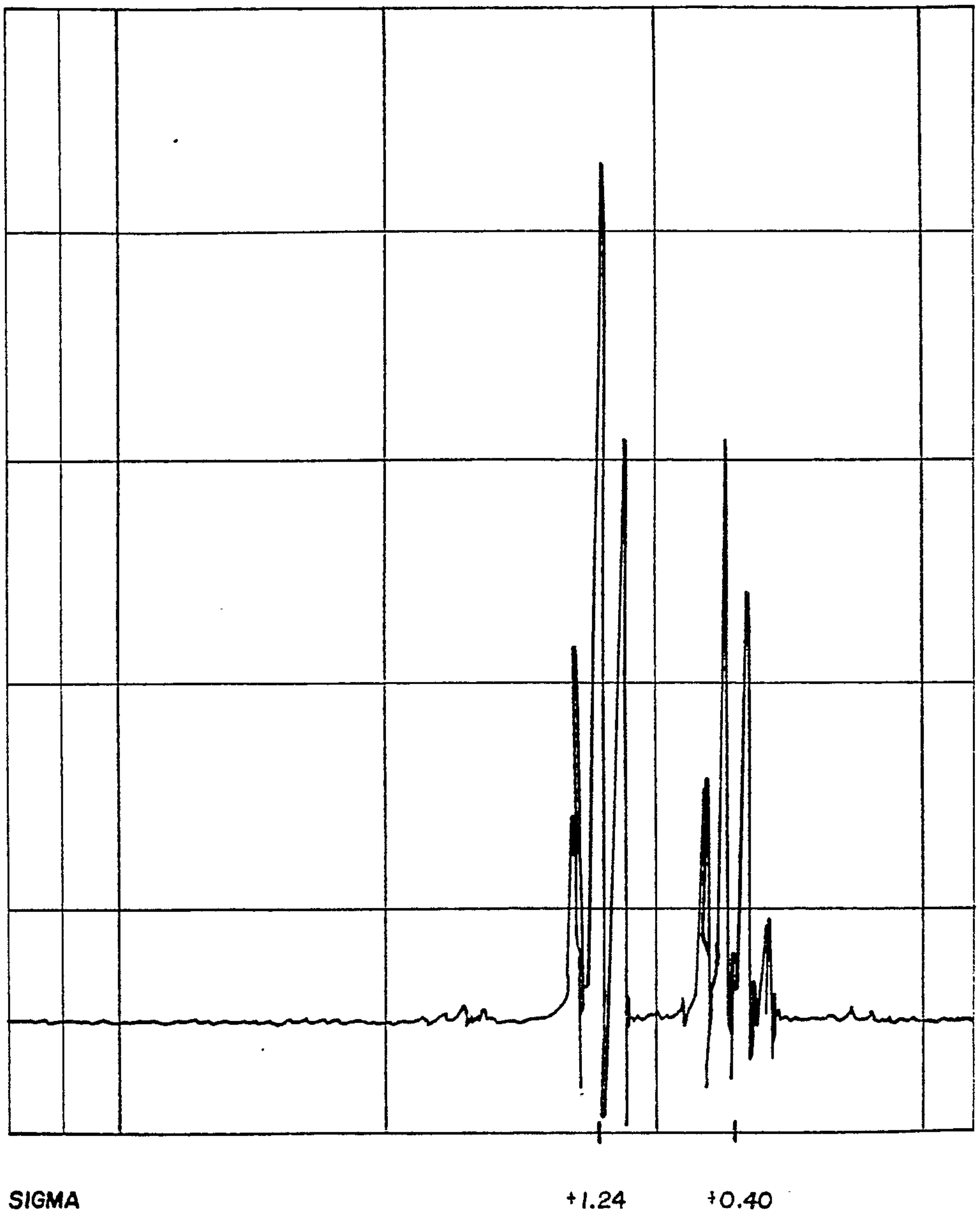


Fig. 6